# FASCICULE DE BREVET EUROPEEN

(11) **EP 0 840 771 B1**
(45) Date de publication et mention de la délivrance du brevet: **22.09.1999**
(21) Numéro de dépôt: 96925783.1
(22) Date de dépôt: 12.07.1996
(51) Int. Cl.: C10G 1/10, C08F 8/50

(54) **PROCEDE DE DEGRADATION CONTROLEE DE POLYMERES HYDROCARBONES**
KONTROLIERTES DEGRADIERUNGSVERFAHREN FÜR POLYMERKOHLENWASSERSTOFFE.
METHOD FOR CONTROLLABLY DECOMPOSING HYDROCARBON POLYMERS

(30) Priorité: 13.07.1995 FR 9508552
(43) Date de publication de la demande: 13.05.1998
(73) Titulaire: CPE - LYON - FCR, 69616 Villeurbanne Cédex (FR)
(72) Inventeur: DUFAUD, Véronique, F-69100 Villeurbanne (FR); BASSET, Jean-Marie, F-69100 Villeurbanne (FR)
(74) Mandataire: Obolensky, Michel
(86) Numéro de dépôt international: FR9601092
(87) Numéro de publication internationale: WO9703147

(56) Documents cités:
- US-A- 5 162 446

## Description

La présente invention se rapporte à un nouveau procédé catalytique conduisant à la dégradation contrôlée de polymères hydrocarbonés sous hydrogène en produits valorisables tels que le méthane et les alcanes inférieurs, les alcanes supérieurs ou tout type d'hydrocarbures, ou des oligomères ou des polymères inférieurs allant dans une gamme très large de poids moléculaire. Des polymères qui peuvent être dégradés par ce nouveau procédé sont des polymères ayant des chaînes à liaison carbone-carbone, par exemple le polyéthylène, le polypropylène ou le polystyrène.

Parmi les domaines d'application de l'invention, on peut mentionner la protection de l'environnement ou l'industrie de production de polymères, notamment de polymères à masse moléculaire contrôlée, les produits pétrochimiques, les carburants (coupes d'hydrocarbures), les lubrifiants ou encore l'énergie. L'invention peut aussi trouver des applications dans l'industrie de l'emballage pour la nourriture, ou tout autre type d'emballage pour lequel les déchets de polymères produits lors de la fabrication ne peuvent être réutilisés.

Une protection accrue de l'environnement requiert la mise en place de nouvelles techniques fiables de traitement des déchets polymères communément appelés plastiques. Parmi les diverses méthodes envisagées pour la réutilisation ou l'élimination des polymères usagés, les recyclages énergétiques et/ou chimiques peuvent apporter des solutions satisfaisantes et complémentaires au recyclage de la matière.

Parmi les solutions concernant les déchets plastiques, l'incinération avec ou sans récupération d'énergie constitue une voie possible mais les températures nécessaires pour ce type de traitement sont très élevées de l'ordre de 400 à 700°C. Ce type de procédé ne peut conduire cependant qu'à une valorisation énergétique des polymères, et présente l'inconvénient de ne pas être écologique car il augmenterait l'effet de serre.

Le recyclage chimique commence à occuper une place intéressante. Cette technique consiste à décomposer chimiquement des macromolécules en vue d'obtenir des molécules de faibles masses moléculaires pouvant être réutilisées pour leurs propriétés physiques ou chimiques ou énergétiques (carburants). D'une manière générale, la dégradation catalytique (craquage ou hydrocraquage) des polyoléfines permet d'améliorer la sélectivité de ce type de réaction par rapport aux processus purement chimiques et thermiques, et par conséquent d'obtenir directement des coupes d'hydrocarbures plus faciles à valoriser. Les catalyseurs de craquage et d'hydrocraquage du polypropylène ou du polyéthylène sont en général des silices, des silices-alumines, des zéolithes ou des catalyseurs à base de cobalt et de molybdène qui fonctionnent à température élevée typiquement entre 200 et 600°C et le plus souvent vers 400°C et sous pression élevée d'hydrogène. Ces techniques sont décrites notamment par BELTRAME et coll. dans Polymer dégradation and stability, **26**, (1989) 29-220., HIROTA et coll. dans Makromol. Chem. Macromol. Symp. 57, 161-173 (1992), Y. ISHIRA et coll. Fuel, **69**, 978, (1990). I. KORFF et coll. dans Erdöl Ergas **105**, (1989) 223.

US-A-5 162 446 décrit un procédé pour la transformation par hydrocaraquage contrôlé en présence d'un catalyseur de polymères issus de monomères diéniques. Le atalyseur est un complexe organique bis(cyclopentadienyl) de zirconium. Le dit complexe n'est pas supporté.

La présente invention a pour but de proposer un nouveau procédé catalytique d'hydrocraquage de polymères de type polyoléfines, susceptible d'être mis en oeuvre dans des conditions moins sévères que les procédés connus jusqu'à présent, pour produire facilement et efficacement avec un matériel simplifié des produits valorisables, d'applications variées en fonction de leur masse moléculaire.

La présente invention a ainsi pour objet un procédé pour la transformation d'un polymère ou d'un oligomère à squelette hydrocarboné dérivé d'un monomère à insaturation éthylénique, en alcanes ou en coupe d'hydrocarbures ou en coupe d'oligomères inférieurs par hydrocraquage contrôlé, caractérisé en ce qu'il comprend les étapes consistant à :
- mettre ledit polymère ou oligomère en présence d'un catalyseur à base d'un hydrure de métal ou d'un complexe hydrure de métaux pouvant être généré in situ à partir d'un complexe organo-métallique hydrocarboné, ledit hydrure de métal ou complexe hydrure de métaux étant supporté sur un support minéral avec ledit support minéral étant un solide divisé comprenant un oxyde métallique ou un mélange d'oxydes métalliques, ledit complexe comportant au moins un ligand hydrocarboné et au moins un ligand hydrure, puis
- faire réagir ce mélange avec de l'hydrogène, réalisant ainsi l'hydrocraquage catalytique dudit polymère ou oligomère.

Selon la présente invention, l'expression "alcanes ou coupe d'hydrocarbures ou coupe d'oligomères inférieurs" signifie des composés hydrocarbonés dont la chaîne comporte un nombre d'atomes de carbone inférieur au nombre d'atomes de carbones du polymère ou oligomère de départ.

Grâce à un catalyseur très performant, il est possible de réaliser selon l'invention l'hydrocraquage des polyoléfines telles que le polyéthylène, le polypropylène, le polystyrène ou le polyisoprène, dans des conditions de température et de pression remarquablement basses par rapport à celles habituellement requises dans l'art antérieur pour des réactions mettant en oeuvre des matière solides macromoléculaires basses conditions dans lesquelles les catalyseurs classiques d'hydrocraquage (silice, silice-alumine, zéolithes) sont inactifs. Le procédé peut ainsi être mis en oeuvre avec un appareillage simplifié dans des conditions de sécurité améliorées. Mais l'originalité de la présente invention réside surtout en la grande versatilité des conditions opératoires du procédé qui seront décrites par la suite, permettant d'imposer la sélectivité de formation de certains produits de dégradation, c'est-à-dire de réaliser une dégradation contrôlée des macromolécules.

En ce qui concerne les polymères, la présente invention permet notamment la dégradation contrôlée de polyoléfines pour donner des polymères à propriétés spécifiques plus intéressantes par suite de la modification des masses moléculaires. Cette dégradation contrôlée peut aussi être réalisée au cours du procédé de polymérisation mais plus généralement après la polymérisation, c'est à dire, lors de la mise en forme du polymère.

De manière générale, les polymères dégradables suivant l'invention peuvent dériver de monomères à une seule insaturation éthylénique polycondensable, soit de type oléfinique tels que l'éthylène, le propylène, l'isobutylène, le méthylpentène, l'hexène, soit de type alcényle aromatique tels que le styrène, les vinylpyridines, ou les vinylnaphtalènes, et/ou de monomères à plusieurs insaturations éthyléniques polycondensables, tels que le butadiène, l'isoprène, le pipérylène, le méthylpentadiène, le phénylbutadiène, les diméthylhexadiènes, les diméthyloctadiènes.

Le procédé de l'invention s'applique plus avantageusement aux polymères dont le squelette hydrocarboné est une polyoléfine saturée, éventuellement substituée, qui dérivent d'un ou plusieurs monomères comportant une seule insaturation éthylénique.

Ces polymères tels que le polyéthylène ou le polypropylène, sont connus pour être d'une stabilité thermique et chimique exemplaire. C'est cette extraordinaire stabilité chimique qui a fait leur succès. C'est malheureusement cette stabilité thermique et chimique qui rend le problème écologique posé par les déchets de ces polymères particulièrement difficile à résoudre. L'invention décrite ici donne des résultats particulièrement spectaculaires en ce qui concerne les conditions d'hydrocraquage des polyoléfines saturées, notamment du polypropylène et du polyéthylène.

L'invention s'applique également aux polymères dont le squelette hydrocarboné, éventuellement substitué, comporte des insaturations, et qui sont issus d'un monomère comportant plusieurs insaturations éthyléniques. Ces polymères insaturés sont en général plus faciles à dégrader que les polymères précédents car leur chaîne est très sensible à la rupture au niveau des insaturations. Le procédé de l'invention est cependant très avantageux sur le plan du contrôle de la dégradation, en ce sens qu'il permet de dégrader également les parties saturées de la chaîne polymère pour réduire cette dernière en petites unités.

L'invention s'applique également avantageusement aux copolymères dont le squelette hydrocarboné est insaturé et qui dérivent d'au moins un monomère à une insaturation éthylénique et au moins un monomère à plusieurs insaturations éthyléniques, ainsi qu'aux mélanges de polymères saturés et insaturés dérivés de tels monomères.

Les catalyseurs qui relèvent de la présente invention sont des catalyseurs à base d'hydrures de métaux ou de complexes, notamment de complexes hydrures, de métaux, de préférence de métaux de transition, supportés sur un support minéral.

Les composés les plus intéressants sont ceux des métaux de transition oxophiles, notamment des métaux des colonnes IIIb, IVb, Vb, et VIb du tableau de classification périodique des éléments, lanthanides et actinides y compris. Les hydrures les plus intéressants sont par exemple les hydrures de scandium, yttrium, lanthane, zirconium, hafnium, titane, tantale, vanadium, niobium, chrome, molybdène, tungstène. Parmi les lanthanides et les actinides on peut citer en particulier le cérium, le lanthane, le néodyme etc...

Par hydrure de métal, au cours de la présente description, on entend un composé inorganique dans lequel le métal est lié à au moins un atome d'hydrogène sous forme hydrure et éventuellement d'autres ligands tels qu'un halogénure. Dans la forme supportée de l'hydrure, le métal peut également être lié à un ou des groupes fonctionnels de la surface du support.

Par complexe organométallique, on comprend tout complexe de coordination dans lequel le métal est lié à au moins un atome de carbone. Selon l'invention, le métal est lié à au moins un ligand carboné, saturé ou non, de préférence en C₁ à C₂₀, lié au métal par au moins un atome de carbone. Il peut s'agir de ligands a tels que alkyle, aryle, carbonyle, acyle ou de ligands π tels que carbène, arène, carbyne, diène, notamment cyclopentadiènyle. Le nombre de ligands dépend du degré d'oxydation du métal qui est typiquement de 2 à 7. Il est préférable qu'au moins un ligand hydrocarboné soit de type σ, de préférence un groupe alkyle ou aryle. On peut citer en particulier les groupes méthyle, éthyle, propyle, isobutyle, néopentyle. De façon très avantageuse, tous les ligands sont de type σ.

Le ou les autres ligands, dont le nombre est fonction du degré d'oxydation du métal, peuvent être de nature variée, notamment halogénure ou oxo.

Dans la forme supportée de ces complexes organométalliques, le métal peut également être lié à un ou des groupes fonctionnels de la surface du support.

Le composé dudit métal peut donc comporter zéro, un ou plusieurs atomes d'hydrogène sous forme hydrure dans sa structure au moment où il est introduit dans le milieu réactionnel. Au cours du cycle catalytique, le métal assure le transfert des atomes d'hydrogène, des molécules H₂ vers les chaînes polymères, en provoquant la rupture de liaisons carbone-carbone. Lorsque le métal est introduit sous forme de complexe hydrocarboné, notamment alkyle, on pense qu'il se forme in situ un complexe hydrure capable d'assurer ce transfert.

Bien que la cause exacte de la réactivité particulière de ces catalyseurs vis-à-vis de l'hydrocraquage des polymères ne soit pas véritablement définie, il semblerait que le support sur lequel est fixé le composé métallique interagisse avec la sphère de coordination du métal pour former une structure hautement réactive.

Le support minéral peut être choisi parmi tous les supports classiquement utilisés pour recevoir un catalyseur d'hydrogénation, notamment les oxydes métalliques ou des chlorures de métaux, ou des sulfures de métaux comme le sulfure de molybdène ou le sulfure de tungstène.

Les supports utilisés dans la présente invention sont de préférence des solides divisés comprenant un oxyde métallique ou un mélange d'oxydes métalliques tels que par exemple la silice-alumine, la silice, l'alumine, les zéolithes, les oxydes mésoporeux, les argiles naturelles, l'oxyde de niobium, la liste n'étant pas limitative. Un des supports préféré est la silice-alumine.

Les supports à base d'oxyde métallique ont l'intérêt de présenter à leur surface des atomes d'oxygène susceptibles de faire partie de la sphère de coordination d'un métal de transition oxophile au sein du composé constituant le catalyseur. La ligandation du métal par des oxygènes de surface confère une plus grande stabilité au composé du métal de transition tout en procurant une liaison support-métal forte.

La préparation des complexes organométalliques supportés utilisables selon l'invention peut être réalisée par greffage d'un complexe organométallique de formule MRₙ où M représente le métal, R un ligand hydrocarboné tel que défini précédemment et n un entier non nul fonction de la valence de M.

La préparation des hydrures ou complexes hydrures supportés utilisables selon l'invention peut être réalisée par (i) greffage d'un composé métallique et préférablement d'un complexe organométallique sur le support minéral en faisant réagir le support avec ledit composé, suivi (ii) d'un traitement sous hydrogène du produit de l'étape (i) destiné à convertir le composé métallique ou organométallique en hydrure dudit métal. En ce qui concerne les composés métalliques ou organométalliques, tous les composés susceptibles d'être réduits en présence d'hydrogène avec formation d'hydrure sont utilisables. Ils répondent de préférence à la formule (1)

MHₓR_{y} (1)

où
M représente le métal,
H représente un ion hydrure,
x est un entier qui peut être nul,
R représente un ligand hydrocarboné,et
y est un entier non nul.

Si y vaut plus de 1, les ligands R peuvent être identiques ou différents.

x peut être nul, le composé greffé étant alors un composé organométallique ne contenant pas d'hydrure, ou x peut valoir au moins 1, le composé greffé comportant alors déjà au moins un ion hydrure.

Le ligand R peut être un ligand carboné quelconque susceptible de former un composé ou un complexe organométallique avec le métal de transition M.

La chaîne carbonée de R comporte avantageusement de 1 à 20 atomes de carbone.

R peut être choisi notamment parmi des ligands alkyle, carbène, carbyne, carbonyle, diène éventuellement substitué, aryle, acyle et arène.

De préférence, au moins l'un des ligands R est un ligand de type σ, avantageusement un groupe alkyle ou aryle.

Lorsque M est un métal des colonnes IIIb, y compris les lanthanides et les actinides, et IVb, par exemple le zirconium, R est avantageusement choisi parmi les groupes alkyle et aryle.

Lorsque M est un métal de transition autre que ceux ci-dessus, R peut avoir d'autres significations. On peut citer par exemple des complexes alkyl-carbène-métal.

Les valeurs de x et de y sont dépendantes du degré d'oxydation du métal dans le composé que l'on veut mettre en oeuvre. En général le métal est à un degré d'oxydation élevé mais il peut dans d'autres cas avoir un bas degré d'oxydation.

Alternativement, le complexe peut déjà contenir au moins un atome d'oxygène et être un complexe de type oxo répondant notamment à la formule (2)

M(O)ₙHₚR_{q} (2)

ou de type alcoxo répondant notamment à la formule (3)

M(OR')ₙHₚR_{q}

où
R et H ont la même signification que précédemment,
n et q sont des entiers non nuls, et
p est un entier qui peut être nul.
R' représente un groupe alkyle, de préférence en C₁-C₁₀.

De tels complexes peuvent être formés avec des métaux tels que le vanadium, le niobium ou le tantale, et répondent par exemple à la formule MOR₃.

On remarquera que lorsque le catalyseur est introduit dans le milieu réactionnel sous forme de complexe hydrocarboné, l'étape (ii) décrite ci-dessus intervient lorsque l'on fait réagir le mélange polymère-catalyseur avec l'hydrogène.

Lorsque le support minéral utilisé est un oxyde métallique, le traitement sous hydrogène d'un composé de métal de transition une fois greffé amène le métal oxophile à accomoder un nombre important d'atomes d'oxygène de la surface, pour conduire à une sphère de coordination simple dans laquelle le métal est à la fois lié à un ou quelques oxygènes de la surface et à un ou plusieurs atomes d'hydrogène sous forme hydrure. Le métal est alors lié à la surface de façon covalente et très forte et ne peut pas être retrouvé dans les produits de réaction. A titre d'exemple il a été trouvé que le monohydrure de zirconium lié à la surface de la silice était lié à 3 atomes d'oxygène de la silice avec une distance de 1,94 Angström et à un quatrième atome d'oxygène distant de 2,6 Angström.

Par exemple, un des complexes préféré, le tétranéopentyl zirconium (ZrNp₄) va réagir avec la surface d'une silice-alumine ou d'une silice selon deux modes possibles :
- Si la silice-alumine ou la silice sont prétraitées entre 200 et 500°C avant le greffage, on obtient un complexe de surface qui répond à la formule (≡Si-O)_{α} Zr Np_{β}. C'est ce complexe de surface qui, après traitement sous hydrogène, va conduire au catalyseur actif pour la présente invention.
- Si la silice-alumine ou la silice sont prétraitées au delà de 600°C avant le greffage, on obtient un complexe de surface qui répond à la formule (≡Si-O)_{α}ZrNp_{β}, ≡Si-Np. C'est ce complexe qui, traité sous hydrogène, va conduire aussi à un catalyseur actif pour la présente invention.

Le traitement sous hydrogène (ii) est de préférence effectué à une température de 50 à 250°C.

La quantité d'hydrogène utilisée dépend de la facilité de réduction du composé greffé. La pression d'hydrogène à cette étape est avantageusement d'environ 10⁵ Pa.

Parmi les supports minéraux susceptibles de stabiliser le catalyseur sous forme hydrure, introduit tel quel ou formé in situ, on peut mentionner tout support oxyde traditionnel par exemple la silice-alumine, la silice, l'alumine, les zéolithes, l'oxyde de titane, l'oxyde de niobium, l'oxyde de magnesium, les supports de type sulfure métalliques, notamment les sulfures de molybdène ou de tungstène, les alumines sulfurées, les oxydes sulfurés. Le type de support peut avoir une influence sur la sélectivité de l'hydrocraquage catalytique des polymères selon l'invention et contribuer à déterminer la distribution des produits que l'on veut obtenir. Dans certains cas le support préféré est la silice-alumine. Mais la silice ou d'autres oxydes conduisent également à des catalyseurs actifs ; la liste n'est pas limitative.

Le procédé de l'invention permet de réaliser la dégradation par hydrocraquage de produits de condensation dérivés d'un monomère à insaturation éthylénique qui ont une masse moléculaire relativement élevée.

Il procure des résultats remarquablement efficaces pour les macromolécules appelées aussi produits de condensation de type polymère ou oligomère possédant une chaîne carbonée comprenant au moins 10 atomes de carbone.

Les polymères traités peuvent être de tout type, syndiotactique, isotactique, atactique, de faible ou de haute masse moléculaire élastomère ou non.

Dans le cas du polyéthylène, on peut traiter aussi bien du polyéthylène de basse ou de haute densité, de faible ou de haute masse moléculaire.

Une des particularités de la présente invention est qu'elle s'applique aux macromolécules de type polymères ou oligomères dont les propriétés physiques sont radicalement différentes de celles des molécules. En général, dans le processus classique d'hydrocraquage des coupes pétrolières, on procède à la coupure catalytique de molécules d'alcanes ou d'oléfines qui ont été transformées en gaz à haute température.

Dans le cas de la présente invention, qui concerne des macromolécules ou polymères, les propriétés physiques sont très différentes, on ne rencontre pas de molécules gazeuses dans le produit de départ. Au contraire, le polymère qui va être dégradé est soit solide, soit fondu, soit en suspension.

Le procédé de l'invention permet d'abaisser les conditions de température et de pression d'hydrogène par rapport aux conditions des procédés catalytiques actuellement appliquées sur les macromolécules jusqu'à des niveaux moins contraignants en matière d'appareillage et moins susceptibles de dégrader le catalyseur.

Avantageusement, l'hydrocraquage catalytique peut avoir lieu à une température de 25 à 300°C. L'abaissement de la température de réaction d'au moins 100°C par rapport aux procédés usuels représente un gain considérable dans le bilan énergétique du procédé.

De même, l'hydrocraquage catalytique est avantageusement conduit sous une pression d'hydrogène de 10³ à 5.10⁷ Pa. Le procédé de l'invention est particulièrement intéressant car on peut réaliser la dégradation du polymère sous relativement faible pression d'hydrogène. Toutefois, selon ses besoins, le spécialiste pourra être amené à travailler sous relativement forte pression d'hydrogène en obtenant des résultats tout aussi intéressants.

Selon les besoins, l'hydrocraquage catalytique peut être plus ou moins long, notamment il peut durer de 30 min à 150 h.

Le spécialiste est à même, en fonction de ses besoins, de faire jouer ces paramètres opératoires, ainsi que notamment la nature du catalyseur, dans ces gammes avantageuses ou bien en-deçà ou au-delà des limites avantageuses indiquées pour imposer une sélectivité particulière à la réaction.

C'est ainsi que l'on ne peut indiquer des conditions opératoires préférées dans l'absolu. L'intérêt du procédé de l'invention est qu'il est entièrement adaptable en fonction du polymère à dégrader et du produit souhaité comme résidu valorisable de dégradation.

Lorsque le polymère est mis en présence du catalyseur dans les conditions de température et de pression d'hydrogène souhaitées, on observe la dégradation plus ou moins rapide du polymère en oligomères et en hydrocarbures de type alcanes allant du méthane jusqu'aux alcanes de poids moléculaire élevé. Le degré de conversion de plus en plus élevé va conduire à la formation de plus en plus élevée d'alcanes légers, les produits finaux étant généralement le méthane, l'éthane et les alcanes légers. Mais l'on peut choisir les conditions réactionnelles pour pouvoir obtenir des oligomères ou des coupes d'hydrocarbures particulièrement intéressantes comme des coupes diesels, des coupes d'essence, de carburant pour automobiles ou pour avion jet, ou des alpha oléfines servant dans le domaine des détergents.

Les conditions de mise en oeuvre de la réaction sont particulièrement versatiles et donc intéressantes et spectaculaires puisque :
- la réaction peut être réalisée dans un solvant dans lequel le polymère peut être mis en suspension. Les solvants les plus intéressants sont les alcanes cycliques comme la décaline ou le cyclopentane, ou tout autre solvant inerte, y compris les solvants fluorés.
- ou bien la réaction peut être mise en oeuvre selon un système solide-solide, c'est à dire que le catalyseur qui est un solide peut être mis au contact du polymère qui est un solide et la réaction se produit à l'interface solide-solide, le polymère étant de préférence sous la forme fondue, sans que cela constitue une nécessité.

La réaction peut également s'appliquer à un mélange de polymères, par exemple en présence simultanée de polypropylène et de polyéthylène ou de polyéthylène, polypropylène et polystyrène dans le réacteur, cela étant particulièrement intéressant pour des finalités écologiques, dans le cas du retraitement univoque de déchets complexes.

Le protocole opératoire est simple :
- en production discontinue, le polymère à dégrader peut être introduit dans un réacteur de type "batch" contenant le catalyseur à base d'hydrure métallique sous atmosphère inerte, de préférence anhydre. Une quantité d'hydrogène est alors introduite dans le réacteur et la température est ajustée à la valeur désirée.
- alternativement, la réaction peut être réalisée en discontinu dans un solvant de type alcane cyclique tel que la décaline ou le cyclopentane. Dans ce cas, la vitesse de réaction est moins rapide mais il est possible d'avoir un meilleur contrôle de la cinétique de la réaction et donc des produits que l'on veut obtenir.
- alternativement, la réaction peut être conduite sous un régime dynamique dans un réacteur adéquat et le catalyseur, le gaz hydrogène et le polymère peuvent être envoyés en continu dans le réacteur.
- alternativement, le polymère en suspension dans un solvant peut être envoyé sur le lit catalytique traversé avec une pression partielle d'hydrogène adéquate permettant d'obtenir le produit ou la coupe de produits réactionnels recherchés.
- alternativement, le polymère peut être modifié au cours de sa mise en forme, par exemple dans une extrudeuse, et c'est au cours de cette mise en forme que la modification a lieu par introduction simultanée du catalyseur, du polymère et de l'hydrogène via des moyens appropriés d'introduction de catalyseur, de polymère et d'hydrogène.

En variante, le procédé de l'invention peut être mis en oeuvre au cours même de la formation dudit polymère ou oligomère, l'hydrocraquage catalytique étant effectuée dans le réacteur même de polycondensation du monomère.

La présente invention, et en particulier la versatilité et les multiples intérêts du procédé de l'invention, vont maintenant être illustrés par les exemples ci-dessous qui font référence aux dessins annexés sur lesquels :
- la figure 1 est un chromatogramme obtenu par chromatographie en phase gazeuse (CPG) d'un polymère de polyéthylène de bas poids moléculaire susceptible d'être dégradé par le procédé de l'invention,
- les figures 2 à 5 sont des chromatogrammes obtenus par CPG du produit de la dégradation du polyéthylène de bas poids moléculaire précité par le procédé de l'invention, avec un premier catalyseur, après des durées de traitement variables,
- la figure 6 est un chromatogramme obtenu par CPG du produit de la dégradation du polyéthylène de bas poids moléculaire précité selon le procédé de l'invention, avec un second catalyseur,
- la figure 7 est un chromatogramme obtenu par CPG du produit de dégradation d'un polyéthylène de bas poids moléculaire précité selon le procédé de l'invention en milieu solvant.

### EXEMPLE DE PREPARATION A

Cet exemple illustre la préparation d'un catalyseur de type complexe hydrure de zirconium supporté sur silice-alumine.

Après avoir été calcinés sous un flux continu d'oxygène à 500°C pendant 10 heures, 5 grammes de silice-alumine (surface spécifique = 380 m²/g) contenant 25% d'aluminium ont été introduits dans un réacteur en verre et traités sous vide à 500°C pendant une nuit. Après ce traitement, 617 mg de ZrNp₄ (Np = néopentyl) à greffer sur le support minéral ont été sublimés dans le réacteur, de façon à ce que la silice-alumine ait une teneur en zirconium de 3 %. Après mise sous vide pour éliminer le complexe n'ayant éventuellement pas réagi, le réacteur a été mis sous une pression d'hydrogène de 10⁵ Pa pendant une nuit à 150°C. Durant ce processus, la formation d'hydrure de surface se produit qui conduit en spectroscopie infrarouge à l'apparition de bandes ν(Zr-H) aux environs de 1642 cm⁻¹. Simultanément on observe en spectroscopie infrarouge l'apparition de bandes ν(Si-H) situées vers 2262 et 2198 cm⁻¹. L'apparition simultanée des bandes Zr-H et Si-H est une preuve de la synthèse du bon catalyseur dans le cadre de l'invention. La présence de bandes Si-H indique que le réseau de la silice-alumine a été profondément modifié par suite du greffage et surtout de la réduction du complexe greffé. Nous appelerons par la suite ce catalyseur le catalyseur A.

### EXEMPLE DE PREPARATION B.

Cet exemple illustre la préparation d'un catalyseur de type complexe hydrure de zirconium supporté sur silice .

Dans un réacteur en verre, 5 grammes de silice (surface spécifique = 200 m²/g) ont été traités sous vide à 500°C pendant une nuit. Après ce traitement, 617 mg de ZrNp₄ à greffer sur le support minéral ont été sublimés dans le réacteur, de façon à ce que la silice ait une teneur en zirconium de 3 %. Après mise sous vide pour éliminer le complexe n'ayant éventuellement pas réagi, le réacteur a été mis sous une pression d'hydrogène de 10⁵ Pa pendant une nuit à 150°C. Durant ce processus, la formation d'hydrure de surface se produit qui conduit en spectroscopie infrarouge à l'apparition de bandes ν(Zr-H) aux environs de 1649 cm⁻¹. Simultanément on observe en spectroscopie infrarouge l'apparition de bandes ν(Si-H) situées vers 2253 et 2191 cm^{-1.} L'apparition des bandes Zr-H et Si-H est une preuve de la synthèse du bon catalyseur dans le cadre de l'invention. La présence de bandes Si-H indique que le réseau de la silice a été profondément modifié par suite du greffage et surtout de la réduction du complexe greffé.
Nous appelerons par la suite ce catalyseur le catalyseur B .

### EXEMPLE 1

Cet exemple illustre la dégradation d'un polymère de type polyéthylène de bas poids moléculaire selon le procédé de l'invention.

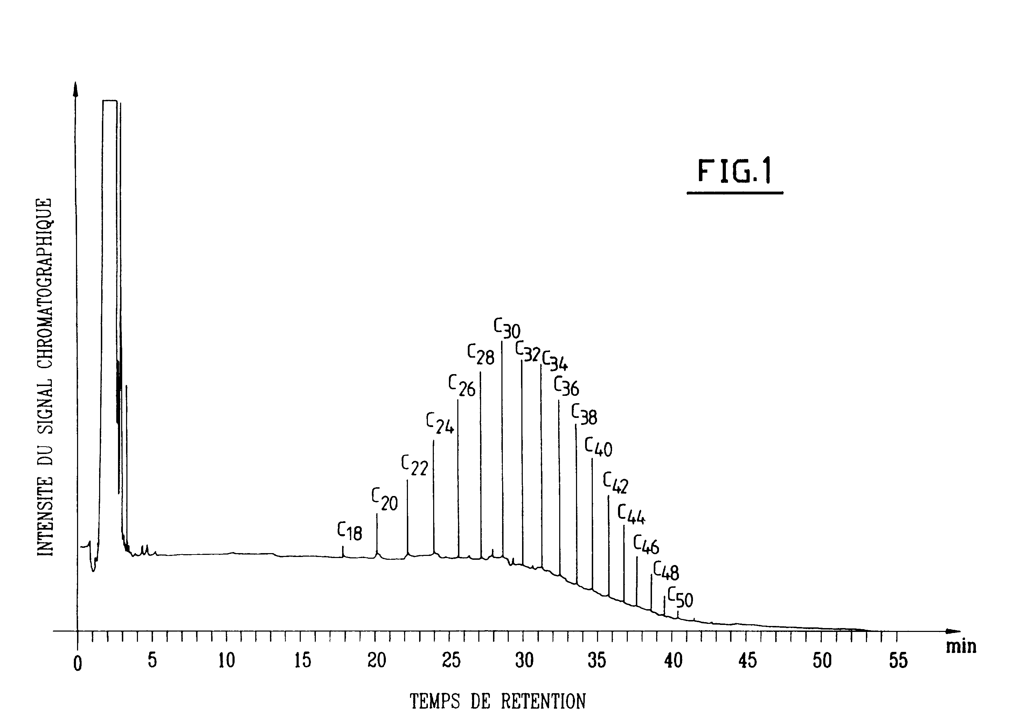

Ce type de polyéthylène comporte une distribution de masses moléculaires assez large allant environ de C₁₈ à C₆₀ . La distribution des oligomères selon le nombre de carbones dans leur chaîne, déterminée par chromatographie en phase gazeuse est présentée sur la figure 1. Le chromatogramme, ainsi que tous les autres chromatogrammes présentés, ont été réalisés avec une montée en température du four. La programmation de température est la suivante :
- température initiale 100°C, maintenue pendant 10 min;
- augmentation de 10°C/min jusqu'à 390°C.

La température de l'injecteur et du détecteur est de 400°C.

Sur le chromatogramme de la figure 1, chaque pic correspond à la détection d'un oligomère. Il apparaît que tous les oligomères détectés possèdent un nombre pair d'atomes de carbone en accord avec la structure macromoléculaire du polyéthylène.

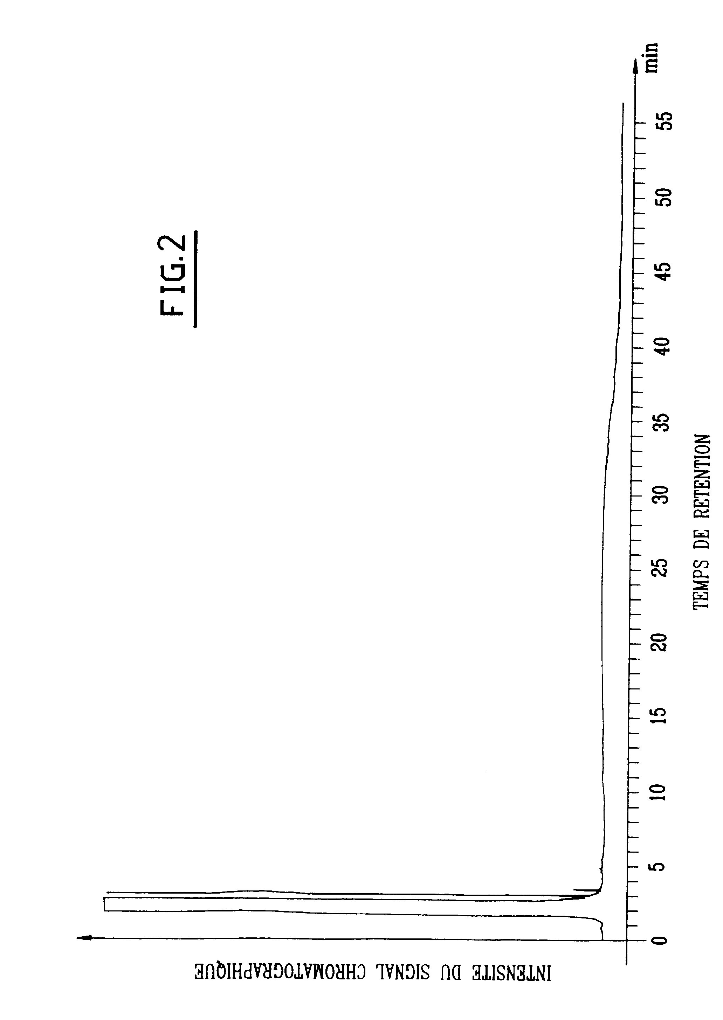

Dans une expérience typique, 70mg du catalyseur A ont été introduits dans un réacteur en verre ayant un volume de 482 ml. La quantité de zirconium correspondante est de 0,023 mmol. Puis 115 mg du polyéthylène ont été introduits dans le réacteur sous atmosphère inerte. La quantité d'atomes de carbone dans les chaînes polymères correspondante est de 8.10⁻³ mol. Enfin une pression d'hydrogène de 10⁵ Pa a été établie dans le réacteur à la température ambiante. La quantité de gaz hydrogène correspondante est de 19,7.10⁻³ mol. Le réacteur a été chauffé à 150°C pendant 62 heures. Les gaz dégagés ont été analysés par chromatographie en phase gazeuse sur une colonne capillaire en silice fondue de type CHROMPACK Al₂O₃/ KCl ; 50 m de longueur . 0.32 mm de diamètre interne. Les hydrocarbures lourds de type oligomères ont été extraits du résidu de réaction dans le réacteur par de la décaline à chaud, puis analysés par CPG (colonne capillaire du type HT5 O.1 mm; 12 m . 0.22 mm recouverte d'alumine) pour conduire au chromatogramme de la figure 2.

Les résultats de l'analyse de la phase gazeuse font apparaître un taux de conversion de 94 (±5)% du polyéthylène de départ en alcanes légers de moins de 9 atomes de carbone. Le chromatogramme de la figure 2, correspondant à l'analyse des hydrocarbures lourds de type oligomères extraits dans la décaline, ne comporte aucun pic dans la gamme des temps de rétention mise en évidence sur la figure 1 et correspondant aux oligomères de départ. Il ne subsiste donc plus de polyéthylène de départ ni d'oligomères de plus faible poids moléculaire. Par contre on note la présence de méthane, éthane, propane, butanes, pentanes et hexanes. Les proportions de ces divers hydrocarbures seront indiquées dans le tableau 1 qui rassemble les résultats comparés des exemples d'application 1 à 4.

A titre comparatif, la même réaction a été effectuée sans hydrure métallique catalytique, mais en présence simplement du support silice-alumine.

70 mg de la silice-alumine utilisée dans l'exemple de référence 1 ont été traités dans les mêmes conditions que dans cet exemple de référence 1, mais sans introduire le complexe de zirconium.

Le même polyéthylène de bas poids moléculaire a été soumis à un traitement sous hydrogène dans les mêmes conditions que celles décrites ci-dessus en remplaçant le catalyseur A par la silice-alumine non greffée. Après 62 heures de réaction, aucune quantité significative de produits de types alcanes légers ni même oligomères n'a pu être détectée.

Cette expérience comparative montre que le support du catalyseur est inactif dans les conditions appliquées et que la dégradation du polyéthylène est rendue possible dans ces conditions par l'hydrure de zirconium.

### EXEMPLE 2

Cet exemple ainsi que les deux exemples qui suivent illustrent le fait que l'on peut règler la nature des produits de la réaction en jouant sur la durée de la réaction.

Le protocole de l'exemple 1 a été exactement reproduit à ceci près que l'on n'a chauffé le réacteur que pendant une heure.

Les résultats de l'analyse de la phase gazeuse font apparaître un taux de conversion de 5 % du polyéthylène de départ en alcanes légers inférieurs à C₉.

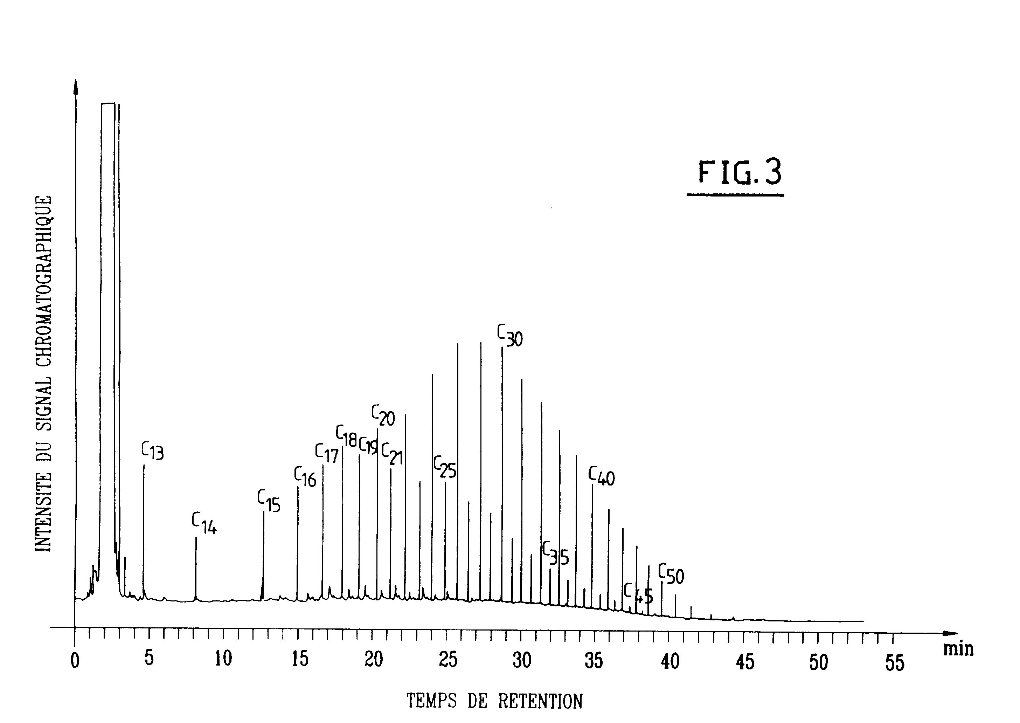

Le chromatogramme de la figure 3, correspondant à l'analyse des hydrocarbures lourds de type oligomères extraits dans la décaline, montre que le polyéthylène de départ a subi des modifications profondes de la distribution des masses moléculaires.

Ainsi, la part des oligomères de plus faible poids moléculaire augmente dans la distribution des oligomères pairs, montrant que la réaction de coupure se produit.

On note en outre l'apparition, entre les pics correspondant aux oligomères de départ, de nouveaux pics correspondant aux oligomères intermédiaires à nombre impair d'atomes de carbone, démontrant que la coupure des liaisons carbone-carbone du polymère de départ est statistique. La distribution des oligomères ayant un nombre de carbone impair atteint son maximum aux environs de C₂₀.

Enfin, en ce qui concerne les hydrocarbures plus légers on note la présence de quantités détectables d'alcanes allant de C₁ à C₇ en particulier de méthane, éthane, propane, butanes, pentanes et hexanes. Les proportions de ces divers hydrocarbures seront indiquées dans le tableau 1.

### EXEMPLE 3

Cet exemple, comme le précédent, illustre le fait que l'on peut régler la nature des produits de la réaction en jouant sur la durée de la réaction.

Le protocole de l'exemple 1 a été exactement reproduit à ceci près que l'on n'a chauffé le réacteur que pendant deux heures.

Les résultats de l'analyse de la phase gazeuse font apparaître un taux de conversion de 25 % du polyéthylène de départ en alcanes légers ayant un nombre de carbone inférieur à C₉.

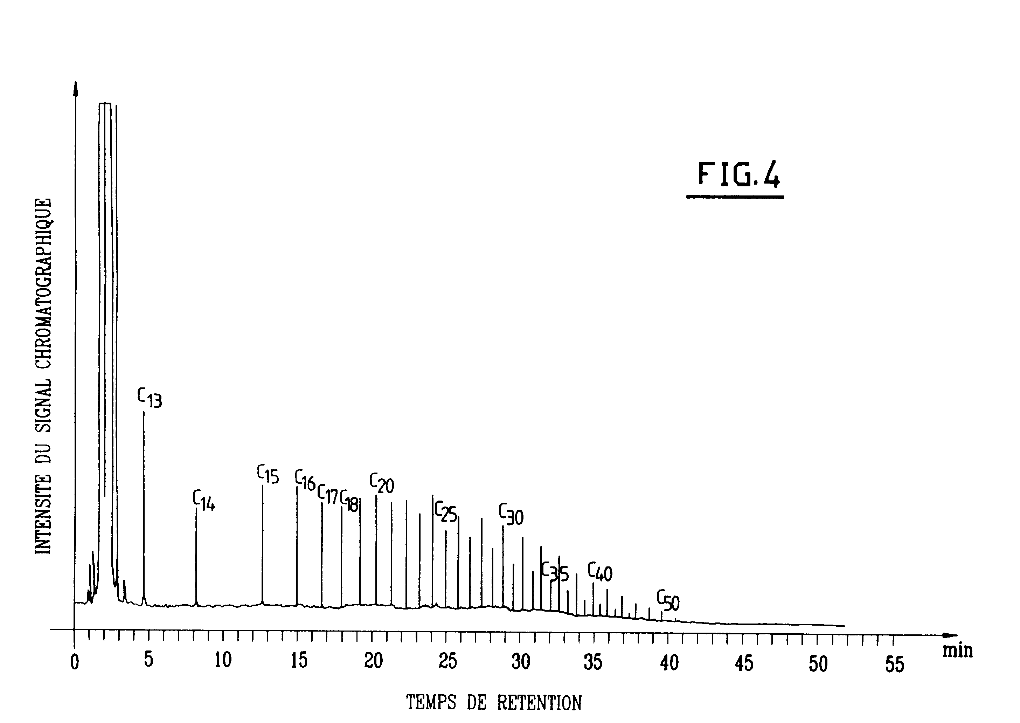

Le chromatogramme de la figure 4, correspondant à l'analyse par CPG des hydrocarbures lourds de type oligomères extraits dans la décaline, montre que le polyéthylène de départ a subi des modifications très profondes de la distribution des masses moléculaires; en particulier on note que des oligomères ayant un nombre de carbone impair apparaissent pratiquement en quantités équivalentes à celle des oligomères à nombre de carbone pair; ce qui fait apparaître une distribution large d'oligomères avec des maxima en nombre de carbone aux environs de C₁₈ et C₂₆.

Enfin en ce qui concerne les hydrocarbures plus légers on note des quantités détectables d'alcanes allant de C₁ à C₈, comme cela est indiqué dans le tableau 1.

### EXEMPLE 4

Cet exemple illustre, comme le précédent, le fait que l'on peut régler la nature des produits de la réaction en jouant sur la durée de la réaction.

Le protocole de l'exemple 1 a été exactement reproduit à ceci près que l'on n'a chauffé le réacteur que pendant cinq heures.

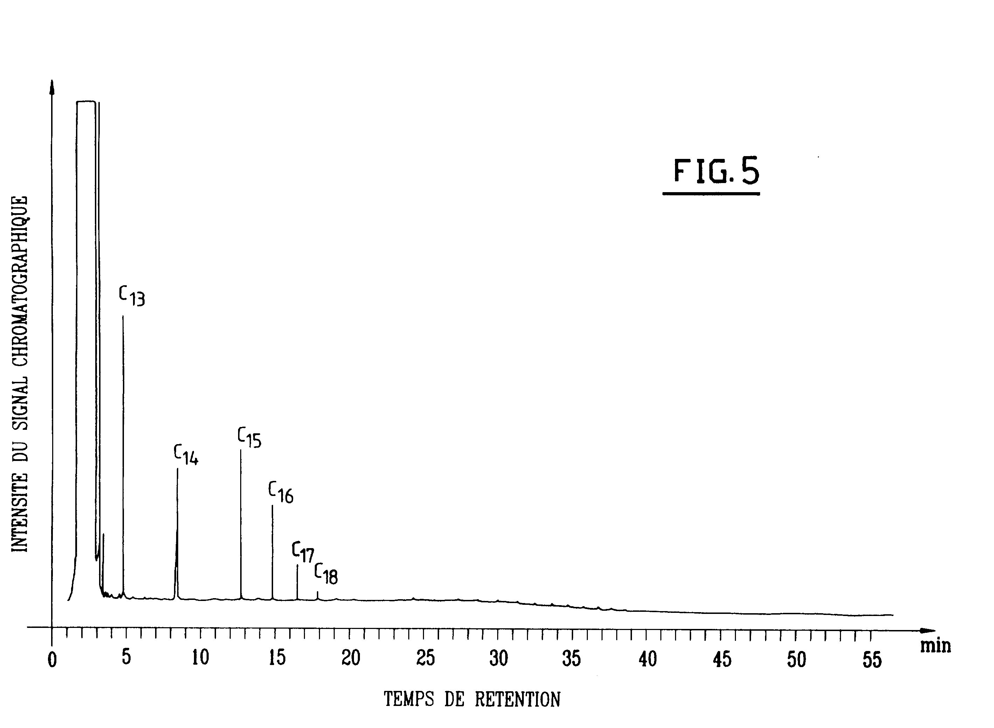

Les résultats de l'analyse de la phase gazeuse font apparaître un taux de conversion de 84 % du polyéthylène de départ en alcanes légers ayant un nombre de carbone inférieur à C₉. Le chromatogramme de la figure 5, correspondant à l'analyse par CPG des hydrocarbures lourds de type oligomères extraits dans la décaline, montre que le polyéthylène de départ a pratiquement disparu. Il ne subsiste qu'une fraction d'hydrocarbures allant de C₁₀ à C₁₈. Enfin en ce qui concerne les hydrocarbures plus légers on note des quantités très importantes d'alcanes allant de C₁ à C₉, qui sont indiqués dans le tableau 1 ci-après.

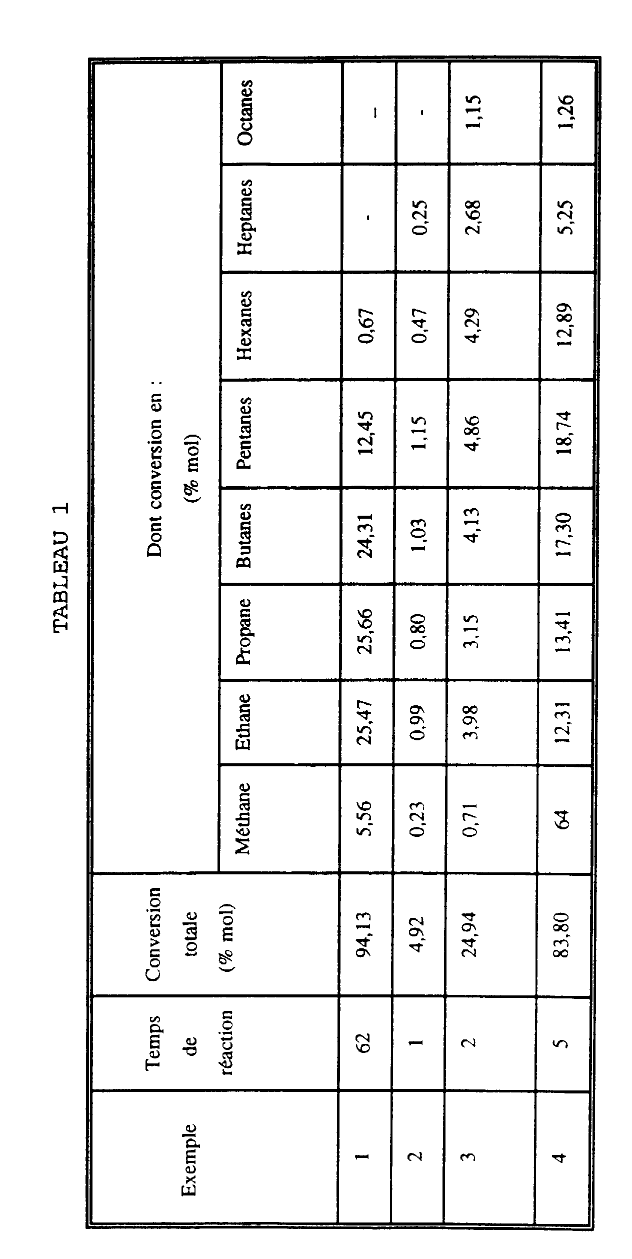

Le tableau 1 montre l'effet de la durée de la réaction sur la distribution des hydrocarbures C₁ à C₈ pour une même quantité de catalyseur, pour une même quantité de polyéthylène, pour une même quantité d'hydrogène et pour une même température.

### EXEMPLE 5

Cet exemple illustre la dégradation du même polymère de polyéthylène catalysée par le catalyseur B.

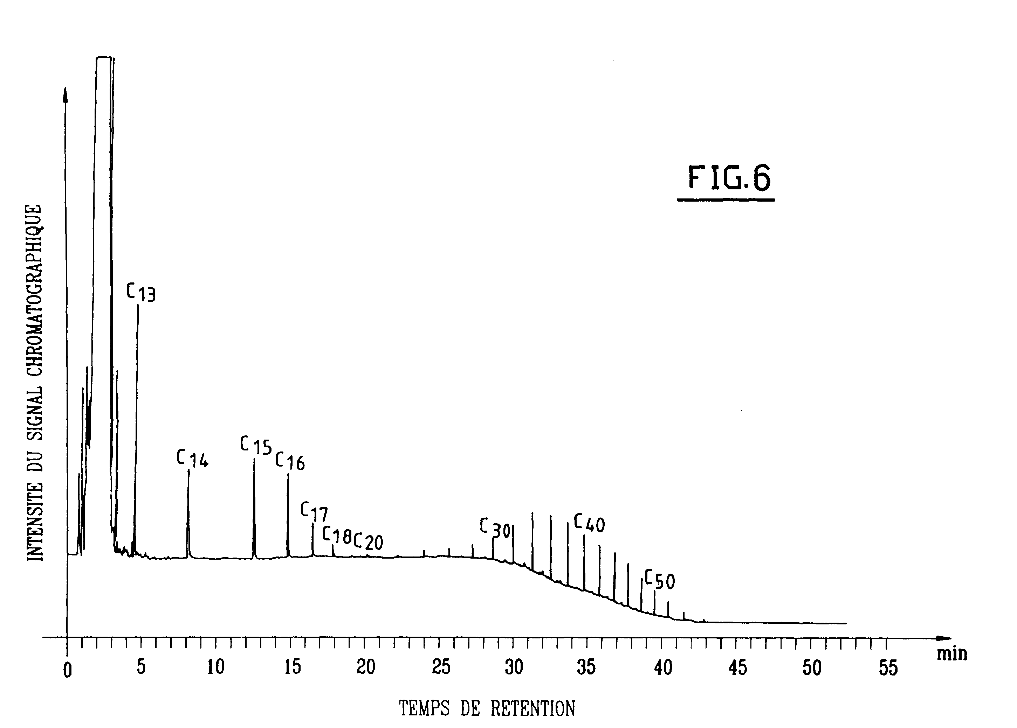

Dans une expérience typique, 60 mg du catalyseur B ont été introduits dans un réacteur en verre ayant un volume de 300 ml. la quantité de zirconium correspondant est de 0,019 mmol. 95 mg de polyéthylène ont été ensuite introduits dans le réacteur sous atmosphère inerte. La quantité d'atomes de carbone dans les chaînes polymères est de 6,8.10⁻³ mol. Enfin une pression d'hydrogène de 8,5.10⁴ Pa a été établie dans le réacteur. La quantité de gaz hydrogène correspondante est de 10,5.10⁻³mol. Le réacteur a été ensuite chauffé à 150°C pendant 30 heures. Les gaz dégagés ont été analysés par chromatographie en phase gazeuse (colonne capillaire en silice fondue de type CHROMPACK Al₂O₃/ KCl ; 50 m de longueur * 0.32 mm de diamètre interne). Les hydrocarbures lourds de type oligomères ont été extraits par la décaline à chaud puis analysés par CPG (colonne capillaire du type HT5 O.1 mm; 12 m de longueur * 0.22 mm de diamètre interne recouverte d'alumine) pour conduire au chromatogramme de la figure 6.

Les résultats de l'analyse de la phase gazeuse font apparaître un taux de conversion de 32% du polyéthylène de départ en alcanes légers inférieurs à C₉, dont la répartition est indiquée dans le tableau 2 ci-après.

La distribution des oligomères apparaissant sur le chromotogramme de la figure 6, correspondant à l'analyse par CPG des hydrocarbures lourds de type oligomères extraits dans la décaline, est singulièrement modifiée, tant par rapport à celle du polymère de départ, que par rapport à celle des produits de dégradation obtenus dans les conditions des exemples d'application 1 à 4 qui précèdent.

On note la formation d'une fraction importante d'hydrocarbures allant de C₁₀ à C₁₈, alors qu'il subsiste une quantité relativement élevée d'oligomères pairs entre C₃₀ et C₅₀.

Dans ce cas, on vérifie également que de la silice prétraitée comme dans l'exemple de référence 2 sans introduction de complexe de zirconium, est inactive pour l'hydrocraquage catalytique du polyéthylène dans les conditions étudiées.

La même expérience comparative peut également être effectuée avec de l'oxyde de niobium traité dans les conditions de l'exemple de référence 1 sans introduction du complexe de zirconium. On vérifie que l'oxyde de niobium ainsi traité est également inactif pour l'hydrocraquage catalytique du polyéthylène dans les conditions des exemples 1 à 5.

**TABLEAU 2**

| | Dont conversion en : (% mol) | | | | | | | |
|---|---|---|---|---|---|---|---|---|
| Conversion totale (%mol) | Méthane | Ethane | Propane | Butanes | Pentanes | Hexanes | Heptanes | Octanes |
| 31,88 | 1,71 | 4,81 | 5,18 | 6,70 | 6,94 | 4,24 | 2,29 | - |

### EXEMPLE 6

Cet exemple illustre l'application du procédé catalytique en phase liquide en utilisant un solvant inerte dans lequel le polymère et le catalyseur se trouvent en suspension.

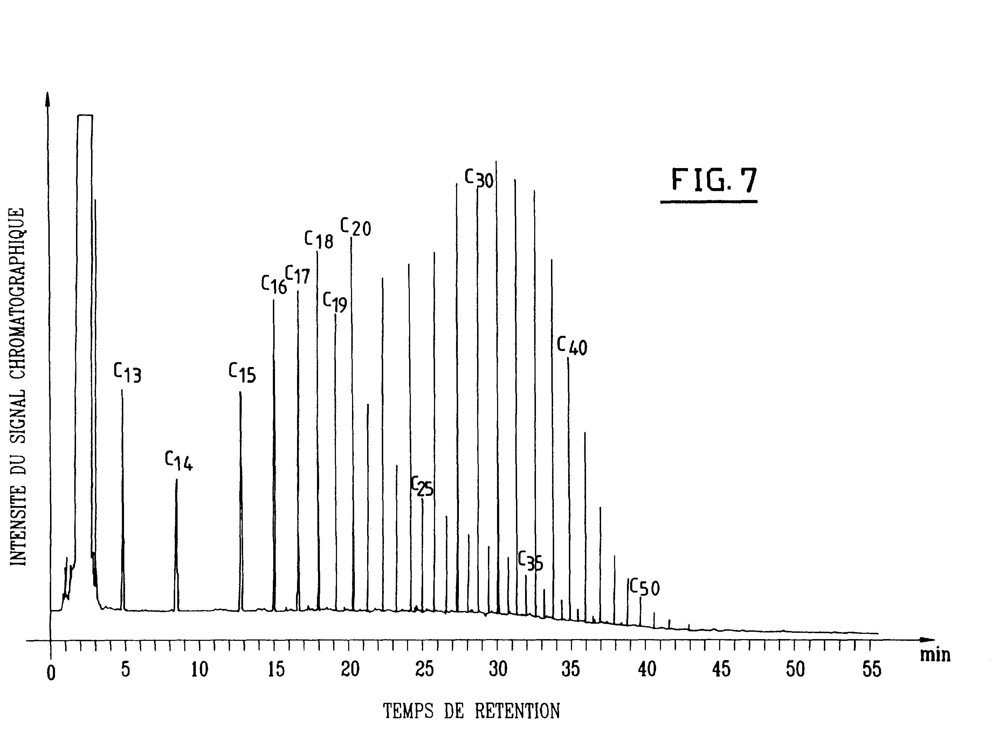

Dans une expérience typique, 70 mg du catalyseur A ont été introduits dans un réacteur en verre ayant un volume de 300 ml. La quantité de zirconium correspondante est de 0,023 mmol. Puis 100 mg de polyéthylène identique à celui utilisé dans les exemples précédents ont été introduits dans le réacteur sous atmosphère inerte. La quantité de carbone dans les chaînes polymères est de 7,4 10⁻³mol. 4 ml de décaline, préalablement séchés, ont été ensuite ajoutés sous atmosphère inerte. Enfin une pression d'hydrogène de 6,5.10⁴ Pa a été établie dans le réacteur. La quantité de gaz hydrogène correspondante est de 8,07 10⁻³ mol. Le réacteur a été ensuite chauffé à 150°C pendant 30 heures. Les gaz dégagés ont été analysés par chromatographie en phase gazeuse (colonne capillaire en silice fondue de type CHROMPACK Al₂O₃/ KCl ; 50 m .0.32 mm) et les hydrocarbures lourds de type oligomères ont été extraits PAR la décaline à chaud puis analysés par CPG (colonne capillaire du type HT5 O.1 mm; 12 m . 0.22 mm recouverte d'alumine) pour fournir le chromatogramme de la figure 7.

L'analyse des hydrocarbures lourds de type oligomères extraits dans la décaline montre que le polyéthylène de départ a subi des modifications profondes de la distribution des masses moléculaires, comme cela apparaît clairement sur la figure 7. Un déplacement de la distribution des oligomères est observé vers les plus faibles masses . De plus, on note l'apparition d'oligomères ayant un nombre de carbone impair démontrant de la même manière que précédemment que la coupure des liaisons carbone-carbone du polymère de départ est statistique. La distribution des oligomères est par conséquent plus large avec des maxima en nombre de carbone aux environs de C₂₀ et C₃₂. L'analyse de la phase gazeuse indique la présence de quantités detectables d'alcanes allant de C₁ à C₇.

On vérifie que tant la silice-alumine, que la silice ou l'oxyde de niobium, lorsqu'ils sont prétraités comme dans les exemples de référence sans introduire de complexe de zirconium, ne sont pas actifs pour l'hydrocraquage du polyéthylène dans ces conditions.

### EXEMPLE 7

Cet exemple illustre l'application du procédé catalytique pour transformer un polypropylène de haut poids moléculaire en alcanes légers et en oligomères de plus faible poids moléculaire.

Dans une expérience typique, 70 mg du catalyseur A ont été introduits dans un réacteur en verre ayant un volume de 300 ml. La quantité de zirconium correspondante est de 0,023 mmole. Puis 136 mg de polypropylène ayant une masse moléculaire moyenne de 250 000 ont été introduits dans le réacteur sous atmosphère inerte. La quantité d'atomes de carbone dans les chaînes polymères est de 9,71 10⁻³ mol. Enfin une pression d'hydrogène de 10⁵ Pa a été établie dans le réacteur. La quantité de gaz hydrogène correspondante est de 12,75 10⁻³ mol. Le réacteur a été ensuite chauffé à 200°C pendant 15 heures.

Les gaz dégagés ont été analysés par chromatographie en phase gazeuse (colonne capillaire en silice fondue de type CHROMPACK Al₂O₃/ KCl ; 50 m * 0.32 mm).

Les résultats de l'analyse de la phase gazeuse font apparaître un taux de conversion de 40% du polypropylène de départ en alcanes lègers inférieurs à C₈, particulièrement en méthane, éthane, propane, butanes, pentanes, hexanes et heptanes, comme cela est indiqué dans le tableau 3 ci-après.

On vérifie que tant la silice-alumine, que la silice ou l'oxyde de niobium, lorsqu'ils sont prétraités comme dans les exemples de référence sans introduire de complexe de zirconium, ne sont pas actifs pour l'hydrocraquage du polypropylène dans ces conditions.

**TABLEAU 3**

| | Dont conversion en % | | | | | | | |
|---|---|---|---|---|---|---|---|---|
| Conversion totale (%) | Méthane | Ethane | Propane | Butanes | Pentanes | Hexanes | Heptanes | Octanes |
| 40,43 | 11,66 | 5,68 | 6,24 | 7,26 | 6,01 | 2,81 | 0,77 | - |

### EXEMPLE 8

Cet exemple illustre l'application du procédé catalytique pour transformer un polystyrène de haut poids moléculaire en alcanes et aromatiques legers (inférieurs à C₆ )et oligomères de plus faible poids moléculaire.

Dans une expérience typique, 75 mg du catalyseur A ont été introduits dans un réacteur en verre ayant un volume de 482 ml. La quantité de zirconium correspondante est de 0,024 mmol. Puis 127 mg de polystyrène (sous forme de billes, mais broyées au préalable avant utilisation) ayant une masse moléculaire moyenne de 45 000 ont été introduits dans le réacteur sous atmosphère inerte. Le nombre de moles d'unité polystyrène -[CH(Ph)CH₂]- dans la chaîne polymère est de 1,22 10⁻³ mol. Enfin une pression d'hydrogène de 10⁵ Pa a été établie dans le réacteur. La quantité de gaz hydrogène correspondante est de 19,7.10⁻³ moles. Le réacteur a été ensuite chauffé à 150°C pendant 15 heures.

Les gaz dégagés ont été analysés par chromatographie en phase gazeuse (colonne capillaire en silice fondue de type CHROMPACK Al₂O₃/ KCl ; 50 m * 0.32 mm) couplée à la spectrométrie de masse.

Les résultats de l'analyse qualitative de la phase gazeuse indiquent que le polystyrène de départ a été dégradé, principalement en benzène.

Des quantités faibles mais détectables d'alcanes légers dont le nombre de carbone est inférieur à 6, particulièrement de méthane, éthane, propanes, butanes, pentanes et hexanes ont été également observées.

On vérifie que tant la silice-alumine, que la silice ou l'oxyde de niobium, lorsqu'ils sont prétraités comme dans les exemples de référence sans introduire de complexe de zirconium, ne sont pas actifs pour l'hydrocraquage du polypropylène dans ces conditions.

### EXEMPLE 9

Cet exemple illustre la préparation d'un catalyseur de type complexe alkyle de zirconium supporté sur silice-alumine et son utilisation dans la dégradation d'un polymère de type polyéthylène de bas poids moléculaire selon le procédé de l'invention.

Après avoir été calcinés sous un flux continu d'oxygène à 500°C pendant 10 heures, 5 g de silice-alumine (surface spécifique = 380 m²/g) contenant 25 % d'aluminium ont été introduits dans un réacteur en verre et traités sous vide dynamique (1,33.10⁻² Pa) à 500°C pendant une nuit.

Après ce traitement, 617 mg de ZrNp₄ (Np = néopentyl) ont été sublimés à 70°C sur le support minéral. La réaction entre le complexe alkyle de zirconium et le support minéral a lieu à 25°C et conduit à la formation d'une espèce majoritaire de surface qui répond à la formule (≡SiO)_{α}ZrNp_{β} et à la libération dans le cas présent de néopentane.

Le complexe ZrNp₄ de départ n'ayant éventuellement pas réagi est séparé à 70°C par mise sous vide dynamique.

Après ce traitement, le support minéral, à savoir la silice-alumine, présente une teneur en zirconium de 3 %.

Dans une expérience d'hydrocraquage typique, 70 mg de ce catalyseur ont été introduits dans un réacteur en verre ayant un volume de 482 ml. La quantité de zirconium correspondante est de 0,023 mmol. Puis 115 mg du polyéthylène ont été introduits dans le réacteur sous atmosphère inerte. La quantité d'atomes de carbone dans les chaînes polymères correspondantes est de 8,10⁻³ mol. Enfin, une pression d'hydrogène de 10⁵ Pa a été établie dans le réacteur à la température ammbiante. La quantité de gaz hydrogène correspondante est de 19,7.10⁻³ mol. Le réacteur a été ensuite chauffé à 150°C pendant 62 heures.

Les résultats de l'analyse de la phase gazeuse montrent un taux de conversion de 94 (± 5) % du polyéthylène de départ en alcanes légers de moins de 9 atomes de carbone. De même, l'analyse des hydrocarbures lourds de type oligomères extraits dans la décaline ne comporte aucun pic dans la gamme des temps de rétention mise en évidence sur la figure 1 et correspondant aux oligomères de départ.

Les résultats sont identiques à ceux obtenus avec le catalyseur A, ce qui confirme que le transfert d'hydrogène au polymère est assuré par le catalyseur alkylezirconium supporté vraisemblablement via un complexe hydrure de surface transitoire, dans les conditions de la réaction de dégradation.

## Revendications

1. Procédé pour la transformation d'un polymère ou d'un oligomère à squelette hydrocarboné dérivé d'un monomère à insaturation éthylénique, en alcanes ou en coupe d'hydrocarbures ou en coupe d'oligomères inférieurs par hydrocraquage contrôlé, caractérisé en ce qu'il comprend les étapes consistant à :
- mettre ledit polymère ou oligomère en présence d'un catalyseur à base d'un hydrure de métal ou d'un complexe hydrure de métaux pouvant être généré in situ à partir d'un complexe organo-métallique hydrocarboné, ledit hydrure de métal ou complexe hydrure de métaux étant supporté sur un support minéral avec ledit support minéral étant un solide divisé comprenant un oxyde métallique ou un mélange d'oxydes métalliques, ledit complexe comportant au moins un ligand hydrocarboné et au moins un ligand hydrure, puis
- faire réagir ce mélange avec de l'hydrogène, réalisant ainsi l'hydrocraquage catalytique dudit polymère ou oligomère.

2. Procédé selon la revendication 1, caractérisé en ce que ledit métal est un métal de transition choisi parmi les métaux des colonnes IIIb, IVb, Vb et Vlb du tableau de classification périodique des éléments y compris lanthanides et actinides.

3. Procédé selon la revendication 2, caractérisé en ce que ledit métal est un métal de transition choisi parmi le scandium, l'yttrium, le lanthane, le zirconium, l'hafnium, le titane, le tantale, le vanadium, le niobium, le chrome, le molybdène et le tungstène.

4. Procédé selon l'une quelconque des revendications précédentes, caractérisé en ce que le support est choisi parmi la silice-alumine, la silice, l'alumine, l'oxyde de niobium et les zéolithes.

5. Procédé selon l'une quelconque des revendications précédentes, caractérisé en ce que le catalyseur comprend un complexe hydrure de métal de transition supporté sur un oxyde métallique divisé.

6. Procédé selon l'une quelconque des revendications précédentes, caractérisé en ce que ledit catalyseur dérive d'un complexe organométallique dans lequel ledit ligand hydrocarboné est choisi parmi des ligands alkyle, carbène, carbyne, carbonyle, diène, aryle, acyle et arène.

7. Procédé selon l'une quelconque des revendications précédentes, caractérisé en ce que ledit catalyseur dérive d'un complexe organométallique comportant au moins un ligand hydrocarboné de type σ.

8. Procédé selon l'une quelconque des revendications précédentes, caractérisé en ce que le catalyseur est à base d'un complexe organométallique comportant au moins un ligand hydrure et préparé en
(i) faisant réagir ledit support avec un composé dudit métal de formule (1,(2)ou (3)
MHₓR_{y} (1)
M(O)ₙHₚR_{q} (2)
M(OR')ₙHₚR_{q} (3)
dans lesquelles
M représente le métal,
H représente un ion hydrure,
x est un entier éventuellement nul,
R représente un ligand hydrocarboné,
y est un entier non nul,
R' représente un groupe alkyle,
n et q sont des entiers non nuls,
p est un entier éventuellement non nul, et
(ii) en soumettant le produit de l'étape (i) à une hydrogénation.

9. Procédé selon la revendication 8, caractérisé en ce que l'étape(ii) est conduite à une température de 50 à 250°C.

10. Procédé selon l'une quelconque des revendications 1 à 9 , caractérisé en ce que ledit polymère ou oligomère possède une chaîne carbonée comprenant au moins 10 atomes de carbone.

11. Procédé selon l'une quelconque des revendications 1 à 10, caractérisé en ce que ledit polymère ou oligomère est un homopolymère, un copolymère ou un mélange de polymères dérivés d'au moins un monomère choisi parmi les monomères oléfiniques à une seule insaturation éthylénique, tels que l'éthylène, le propylène et le styrène, et les monomères à plusieurs insaturations éthyléniques.

12. Procédé selon la revendication 11, caractérisé en ce que le squelette hydrocarboné dudit polymère est une polyoléfine saturée.

13. Procédé selon la revendication 11, caractérisé en ce que le squelette hydrocarboné dudit polymère est insaturé et en ce que ledit polymère est un copolymère d'au moins un monomère à une seule insaturation éthylénique et d'au moins un monomère à plusieurs insaturations éthyléniques.

14. Procédé selon l'une quelconque des revendications précédentes, caractérisé en ce que ledit hydrocraquage catalytique est effectué une température de 25 à 300°C.

15. Procédé selon l'une quelconque des revendications 1 à 14, caractérisé en ce que ledit hydrocraquage catalytique est conduit sous une pression d'hydrogène de 10³ à 5.10⁷ Pa.

16. Procédé selon l'une quelconque des revendications 1 à 14, caractérisé en ce que ledit hydrocraquage catalytique dure de 30 min à 150 h.

17. Procédé selon l'une quelconque des revendications 1 à 14, caractérisé en ce que ledit hydrocraquage catalytique est conduit dans un milieu liquide comprenant un solvant.

18. Procédé selon la revendication 17, caractérisé en ce que le solvant est choisi parmi les alcanes cycliques.

19. Procédé selon l'une quelconque des revendications 1 à 16, caractérisé en ce que ledit hydrocraquage catalytique est conduit en l'absence de solvant.

20. Procédé selon l'une quelconque des revendications 1 à 19, caractérisé en ce qu'il est mis en oeuvre au cours de la formation dudit polymère ou oligomère.

21. Procédé selon l'une quelconque des revendications 1 à 19, caractérisé en ce qu'il est mis en oeuvre au cours de la mise en forme dudit polymère ou oligomère.

## Patentansprüche

1. Verfahren zur Umwandlung/Umsetzung eines Polymers oder eines Oligomers mit einem Koblenwasserstoffgerüst, das von einem ethylenischungesättigten Monomer abstammt, zu Alkanen oder zu Kohlenwasserstoffsegmenten oder zu Segmenten niederer Oligomere durch kontrolliertes Hydrokracken, dadurch gekennzeichnet, daß es die Schritte umfaßt, die aus
Bringen des besagten Polymers oder Oligomers in Gegenwart eines Katalysators auf der Basis eines Metallhydrids oder eines Metallhydridkomplexes, der in situ auf Grundlage eines organo-metallischen Kohlenwasserstoffkomplexes erzeugt werden kann, wobei besagtes Metallhydrid oder besagter Metallhydridkomplex von einem mineralischen Träger getragen wird, wobei besagter mineralischer Träger ein verteilter Feststoff ist, der ein Metalloxid oder ein Gemisch von Metalloxiden umfaßt, besagter Komplex wenigstens einen Kohlenwasserstoffliganden und wenigstens eines Hydridliganden umfaßt, danach
Reagierenlassen dieses Gemisches mit Wasserstoff, wodurch das katalytische Hydrokracken des besagten Polymers oder Oligomers durchgeführt wird, bestehen.

2. Verfahren nach Anspruch 1, dadurch gekennzeichnet, daß besagtes Metall ein Übergangsmetall ist, das aus den Metallen der Gruppen IIIb, IVb, Vb und VIb des Periodensystems der Elemente, einschließlich der Lanthaniden und Aktiniden ausgewählt wird.

3. Verfahren nach Anspruch 2, dadurch gekennzeichnet, daß besagtes Metall ein Übergangsmetall ist, das aus Scandium, Yttrium, Lanthan, Zirkonium, Hafnium, Titan, Tantal, Vanadium, Niob, Chrom, Molybden und Wolfram ausgewählt wird.

4. Verfahren nach einem der vorhergehenden Ansprüche, dadurch gekennzeichnet, daß der Träger aus Siliciumdioxid-Aluminiumoxid, Siliciumdioxid, Aluminiumoxid, Nioboxid und Zeolithen ausgewählt wird.

5. Verfahren nach einem der vorhergehenden Amprüche, dadurch gekennzeichnet, daß der Katalysator einen Übergangsmetallhydridkomplex umfaßt, der von einen verteilten Metalloxid getragen wird.

6. Verfahren nach einem der vorhergehenden Ansprüche, dadurch gekennzeichnet, daß besagter Katalysator von einem organometallischen Komplex abstammt, in dem besagter Kohlenwasserstoffligand aus Alkyl-, Carben-, Carbin-, Carbonyl-, Dien, Aryl-, Acyl und Arenliganden ausgewählt wird.

7. Verfahren nach einem der vorhergehenden Ansprüche, dadurch gekennzeichnet, daß besagter Katalysator von einem organometallischen Komplex abstammt, der wenigstens einen Kohlenwasserstoffliganden vom σ-Typ umfaßt.

8. Verfahren nach einem der vorhergehenden Ansprüche, dadurch gekennzeichnet, daß der Katalysator auf der Basis eines organometallischen Komplexes ist, der wenigstens einen Hydridliganden umfaßt und hergestellt wird, indem
(i) besagter Träger mit einer Verbindung des besagten Metalles der Formel (1), (2) oder (3) zur Reaktion gebracht wird:
MHₓR_{y} (1)
M(O)ₙHₚR_{q} (2)
M(OR')ₙHₚR_{q} (3)
in denen
M das Metall darstellt,
H ein Hydridion darstellt,
x eine ganze Zahl ist, die gegebenenfalls Null ist,
R einen Kohlenwasserstoffliganden darstellt,
y eine ganze Zahl ungleich Null ist,
R' eine Alkylgruppe darstellt,
n und q ganze Zahlen ungleich Null sind,
p eine ganze Zahl ist, die gegebenenfalls nicht Null ist, und
(ii) indem das Produkt von Schritt (i) Hydrierung unterworfen wird.

9. Verfahren nach Anspruch 8, dadurch gekennzeichnet, daß Schritt (ii) bei einer Temperatur von 50 bis 250 °C geführt wird.

10. Verfahren nach einem der Ansprüche 1 bis 9, dadurch gekennzeichnet, daß besagtes Polymer oder Oligomer eine Kohlenstoffkette besitzt, die wenigstens 10 Kohlenstoffatome umfaßt.

11. Verfahren nach einem der Ansprüche 1 bis 10, dadurch gekennzeichnet, daß besagtes Polymer oder Oligomer ein Homopolymer, ein Copolymer oder ein Polymergemisch ist, die von wenigstens einem Monomer abstammen, das aus olefinischen Monomeren mit einer einzigen ethylenischen Ungesättigtheit wie Ethylen, Propylen, Styren und aus Monomeren mit mehreren ethylenischen Ungesättigtheiten ausgewählt wird.

12. Verfahren nach Anspruch 11, dadurch gekennzeichnet, daß das Kohlenwasserstoffgerüst des besagten Polymers ein gesättigtes Polyolefin ist.

13. Verfahren nach Anspruch 11, dadurch gekennzeichnet, daß das Kohlenwasserstoffgerüst des besagten Polymers ungesättigt ist und daß besagtes Polymer ein Copolymer von wenigstens einem Monomer mit einer einzigen ethylenischen Ungesättigtheit und von wenigstens einem Monomer mit mehreren ethylenischen Ungesättigtheiten ist.

14. Verfahren nach einem der vorhergehenden Ansprüche, dadurch gekennzeichnet, daß besagtes katalytisches Hydrokracken bei einer Temperatur von 25 bis 300 °C durchgeführt wird.

15. Verfahren nach einem der Ansprüche 1 bis 14, dadurch gekennzeichnet, daß besagtes katalytisches Hydrokracken unter einem Wasserstoffdruck von 10³ bis 5.10⁷ Pa geführt wird.

16. Verfahren nach einem der Ansprüche 1 bis 14, dadurch gekennzeichnet, daß besagtes katalytisches Hydrokracken 30 min bis 150 h dauert.

17. Verfahren nach einem der Amprüche 1 bis 14, dadurch gekennzeichnet, daß besagtes katalytisches Hydrokracken in einem flüssigen Medium durchgeführt wird, das ein Lösungsmittel umfaßt.

18. Verfahren nach Anspruch 17, dadurch gekennzeichnet, daß das Lösungsmittel aus cyclischen Alkanen ausgewählt wird.

19. Verfahren nach einem der Ansprüche 1 bis 16, dadurch gekennzeichnet, daß besagtes katalytisches Hydrokracken in Abwesenheit eines Lösungsmittels durchgeführt wird.

20. Verfahren nach einem der Ansprüche 1 bis 19, dadurch gekennzeichnet, daß es während der Bildung des besagten Polymers oder Oligomers eingesetzt wird.

21. Verfahren nach einem der Ansprüche 1 bis 19, dadurch gekennzeichnet, daß es während der Verarbeitung des besagten Polymers oder Oligomers eingesetzt wird.

## Claims

1. A process for transforming a polymer or an oligomer with a hydrocarbon backbone derived from an unsaturated ethylenic monomer into alkanes or a lower hydrocarbon cut or a lower oligomer cut, through controlled hydro-cracking, characterized in that it comprises the steps consisting of:
- placing the said polymer or oligomers in the presence of a catalyst based on a metal hydride or a complex hydride of metals adapted to be formed in situ from an organo-metallic hydrocarbon complex, the said metal hydride or complex hydride of metals being supported on a mineral support, the said mineral support being a divided solid comprising a metallic oxide or mixture of metallic oxides, the said complex comprising at least one hydrocarbon ligand and at least one hydride ligand, then
- causing the mixture to react with hydrogen, thus effecting the catalytic hydro-cracking of the said polymer or oligomer.

2. A process according to claim 1, characterized in that the said metal is transition metal selected from the metals of columns IIIb, IVb, Vb and VIb of the periodic table classification of elements including lanthanides and actinides.

3. A process according to claim 2, characterized in that the said metal is a transition metal selected from scandium, yttrium, lanthanum, zirconium, hafnium, titanium, tantalum, vanadium, niobium, chromium, molybdenum and tungsten.

4. A process according to any of the preceding claims, characterized in that the support is selected from silica-alumina, silica, alumina, niobium oxide and the zeolites.

5. A process according to any of the preceding claims, characterized in that the catalyst comprises a complex hydride of a transition metal supported on a divided metal oxide.

6. A process according to any of the preceding claims, characterized in that the said catalyst derives from an organo-metallic complex in which the said hydrocarbon ligand is selected from the alkyl, carbene, carbyne, carbonyl, diene, aryl, acyl and arene ligands.

7. A process according to any of the preceding claims, characterized in that the said catalyst derives from an organo-metallic complex comprising at least one hydrocarbon ligand of a type.

8. A process according to any of the preceding claims, characterized in that the catalyst is based on an organo-metallic complex comprising at least one hydride ligand and prepared by
(i) causing the said support to react with a compound of the said metal of formula (1), (2) or (3)
MHₓR_{y} (1)
M(O)ₙHₚR_{q} (2)
M(OR')ₙHₚR_{q} (3)
in which
M represents the metal,
H represents a hydrogen ion,
x is an integer which may be zero,
R represents a hydrocarbon ligand,
y is a non-zero integer,
R' represents an alkyl group,
n and q are non-zero integers,
p is an integer which may be zero, and
(ii) subjecting the product of step (i) to hydrogenation.

9. A process according to claim 8, characterized in that the step (ii) is performed at a temperature from 50 to 250° C.

10. A process according to any of claims 1 to 9, characterized in that the said polymer or oligomer has a carbon chain comprising at least 10 carbon atoms.

11. A process according to any of claims 1 to 10, characterized in that the said polymer or oligomer is a homo-polymer, a copolymer or a mixture of polymers derived from at least one monomer selected from the olefinic monomers with a single ethylenic unsaturated bond, such as ethylene, propylene and styrene, and the monomers with several ethylenic unsaturated bonds.

12. A process according to claim 11, characterized in that the hydrocarbon backbone of the said polymer is a saturated poly-olefine.

13. A process according to claim 11, characterized in that the hydrocarbon backbone of the said polymer is unsaturated and in that the said polymer is a copolymer of at least one monomer with a single ethlenic unsaturated bond and of at least one monomer with several ethylenic unsaturated bonds.

14. A process according to any of the preceding claims, characterized in that the said catalytic hydro-cracking is effected at a temperature from 25 to 300° C.

15. A process according to any of claims 1 to 14, characterized in that the said catalytic hydro-cracking is effected under a pressure of hydrogen from 10³ to 5.10⁷ Pa.

16. A process according to any of claims 1 to 14, characterized in that the said catalytic hydro-cracking lasts from 30 min to 150 h.

17. A process according to any of claims 1 to 14, characterized in that the said catalytic hydro-cracking is carried out in a liquid medium comprising a solvent.

18. A process according to claim 17, characterized in that the solvent is selected from the cyclic alkanes.

19. A process according to any of claims 1 to 16, characterized in that the said catalytic hydro-cracking is carried in the absence of a solvent.

20. A process according to any of claims 1 to 19, characterized in that it is utilised during the course of formation of the said polymer or oligomer.

21. A process according to any of claims 1 to 19, characterized in that it is utilised during the course of the formation of the said polymer or oligomer.
